# EUROPÄISCHE PATENTANMELDUNG

(11) **EP 0 842 671 A2**
(43) Veröffentlichungstag der Anmeldung: **20.05.1998**
(21) Anmeldenummer: 97118681.2
(22) Anmeldetag: 28.10.1997
(51) Int. Cl.: A61M 5/32, A61M 25/06, A61B 5/14

(54) **Verfahren und Vorrichtung zum Entsorgen der Kanüle einer Blutentnahmevorrichtung**

(30) Priorität: 05.11.1996 DE 19645514
(71) Anmelder: Sarstedt AG & Co., 51582 Nümbrecht (DE)
(72) Erfinder: Sarstedt, Walter, 51582 Nümbrecht (DE); Amokrane, Youcef, 91190 Gyf sur Yvette (FR)
(74) Vertreter: Pollmeier, Felix, Dipl.-Ing.

(57) **Zusammenfassung**

Zum Entsorgen der bei einer Blutentnahmevorrichtung zur Blutentnahme mit einem Gefäß adaptierten Kanüle (1) sieht ein Verfahren vor, daß sich ohne Änderung an den vorhandenen Bauteilen bzw. Systemen von Blutentnahmevorrichtungen mit einfachen Mitteln eine sichere Entsorgung der Kanüle gewährleisten läßt, wenn die Kanüle (1) nach dem Abkoppeln des Gefäßes mit einem in einem Schutzgehäuse (7) axial beweglichen Zugelement (9) gekoppelt und samt ihres Adapters (6) vollständig in das Schutzgehäuse (7) hineingezogen wird. Zu diesem Zweck ist in einem eine größere Länge als die Kanüle (1) besitzenden zylindrischen, zumindest einseitig offenen Schutzrohr (7) ein Kanülenhalterkolben (8) angeordnet, der an seiner einen Seite mit einem zu der Führungshülse (3) der Kanüle (1) komplementären Adapterkopf (6) und an seiner anderen Seite mit einem aus dem rückwärtigen Schutzrohrende herausragenden Zugelement (9) ausgebildet ist.

## Beschreibung

Die Erfindung betrifft ein Verfahren und eine Vorrichtung zum Entsorgen der bei einer Blutentnahmevorrichtung zur Blutentnahme mit einem Gefäß addaptierten Kanüle.

Derartige Blutentnahmevorrichtungen sind beispielsweise durch die DE 29 48 653 C und DE 30 49 503 C bekanntgeworden. Zur Blutentnahme wird auf einen exzentrisch - alternativ mittig - angeordneten Ansatz bzw. Dom, ggf. unter Zwischenschaltung eines Anpassungsstückes, eine zur Adaption mit dem Dom dienende Führungshülse mit einer darin gehaltenen Kanüle aufgesetzt, die in der Ausführung als Doppelkanüle beidendig angeschärfte Schneidkanten aufweist; daneben sind auch Kanülen gebräuchlich, die lediglich ein angeschärftes Ende besitzen. Das aus der Führungshülse vorkragende Kanülenende dient zur Einführung in eine Vene, während das hintere Kanülenende so weit in die Führungshülse hineinragt, daß es beim Ansetzen bzw. Ankoppeln der Führungshülse an das Entnahmeröhrchen den elastischen, in dem Dom bzw. dem Anpassungsstück angeordneten Stopfen durchsticht. Das hintere, in die Führungshülse hineinragende Kanülenende wird von einem sackartigen Schlauch (Ventilgummi) solcher Länge eingehüllt, daß die Schneidkante des hinteren Kanülenendes bei gestrecktem Schlauch noch nicht dessen Boden berührt. Das geschieht erst bei zunehmendem Aufschieben der Führungshülse auf den Dom des Blutgefäßes, wobei die angeschärfte Schneidkante der Kanäle zunächst das Ventilgummi und dann den Stopfen durchbohrt, womit der Blutfluß von einem Blutaufnahmegefäß oder der Vene eines Patienten zum Inneren des durch eine aufgeschraubte oder -gesteckte Kappe verschlossenen Entnahmeröhrchens bzw. Blutgefäßes hergestellt wird; Beim Aufschieben der Doppelkanüle auf den Dom der Verschlußkappe wird das Ventilgummi zieharmonikaartig zusammengedrückt.

Das von der Führungs- bzw. Ankopplungshülse ungeschützte, vordere Kanülenende wird mit einer zur Sicherheit aufgesteckten Schutzkappe angeliefert, die vor dem Gebrauch der Kanüle entfernt werden muß. Um einen Schutz vor Stichverletzungen auch nach dem Gebrauch zu erreichen, wird diese Kappe nach Benutzung durch den Anwender vielfach unerlaubterweise wieder auf das vordere Kanülenende aufgesteckt. Die Schutzkappen besitzen einen sehr geringen Innendurchmesser, und es besteht die große Gefahr, daß das vordere Kanülenende nicht in die Kappe eingefädelt, sondern an ihr vorbei in die Hand des Benutzers geführt wird. Diese Gefährdung besteht insbesondere dann, wenn der Benutzer durch äußere Umstände von dieser eine gewisse Aufmerksamkeit erforndernden Handhabung abgelenkt wird. Um diesem aufgrund des kleinen Durchmessers der Schutzkappe unausweichlichen Nachteil zu begegnen, wurden Vorkehrungen in Form von Vorrichtungen getroffen, die ein um die Einführöffnung der Schutzkappe herum angeordnetes, die gesamte Hand des Benutzers abdeckendes Schild aufweisen (vgl. "Special Report und Product Review", Mai 1991, Vol. 20, No. 5, Seite 171, 172). Hier hat sich jedoch die unförmige und sperrige Handhabung des Schutzschildes als nachteilig herausgestellt.

Bei medizintechnischen Produkten anderer Art, die für ihren Einsatzzweck eine angeschärfte Nadel erfordern, nämlich Injektionsspritzen, ist es bekannt, die Nadel - nachdem die Flüssigkeit injiziert worden ist - an den nach vorne geschobenen Kolben der Injektionsspritze anzukoppeln und die Nadel anschließend in das Innere der leeren Spritze zu ziehen. Abgesehen davon, daß hierzu aufwendige, die gesamte Ausführung erheblich verteuernde Konstruktionen notwendig sind, erfordern diese Ausführungen stets speziell angepaßte Entnahme- bzw. Injektionssysteme (vgl. DE 91 09 584 U, EP 0 430 159 B1 und EP 0 602 882 A2). Ein solches Vorgehen ist bei Injektionsspritzen möglich, da der Spritzenzylinder nach der Injektion der Flüssigkeit verbraucht, d .h. geleert ist. Das ist aber bei den gattungsgemäßen Blutentnahmevorrichtungen nicht der Fall, da die hier zum Einsatz kommenden Blutentnahmeröhrchen bzw- gefäße das Blut aufnehmen und bis zur labormäßigen Untersuchung des den Patienten entnommenen Blutes als Transport -und Lagerbehälter dienen. Bei nach dem Vakuumprinzip arbeitenden Blutentnahmevorrichtungen ist es schließlich noch bekannt, die dort verwendeten Nadel- bzw. Kanülenhalter mit einer zweiten, axial über die Kanüle schiebbaren Schutzhülse auszustatten. Die in der Grundform schon voluminösen Halter werden so in ihren Ausmaßen nahezu verdoppelt.

Der Erfindung liegt die Aufgabe zugrunde, ein Verfahren und eine Vorrichtung der genannten Art zu schaffen, mit denen sich ohne Änderung an den vorhandenen Bauteilen bzw Systemen von Blutentnhamevorrichtungen mit einfachen Mitteln eine sichere Entsorgung der Kanüle gewährleisten läßt.

Diese Aufgabe wird nach der Erfindung verfahrensmäßig dadurch gelöst, daß die Kanüle nach dem Abkoppeln des Gefäßes mit einem in einem Schutzgehäuse axial beweglichen Zugelement gekoppelt und samt ihres Adapters vollständig in das Schutzgehäuse hineingezogen wird. Es läßt sich somit ein Vollschutz der Kanüle erreichen, so daß für den Benutzer keine Gefahr einer Berührung und damit einer Stichverletzung besteht, was vor allem dann zusätzliche Bedeutung gewinnt, wenn mit Infektionen gerechnet werden muß. Das Ankoppeln des Schutzgehäuses an die Kanüle bzw. deren Führungshülse sollte vorteilhaft dann vorgenommen werden, wenn nach der letzten Blutentnahme - an die in die Vene des Patienten eingestochene Kanüle lassen sich ein oder mehrere Blutentnahmeröhrchen bzw. -gefäße ansetzen und füllen - die Kanüle sich noch in der Vene des Patienten befindet. Sobald die Kanüle an das Schutzgehäuse angekoppelt ist, wird sie aus der Vene entfernt und danach vollständig in das Schutzgehäuse hineingezogen. Die Abmessungen des Schutzgehäuses brauchen - im Gegensatz zu den bekannten, axial über die Kanüle schiebbaren Schutzhülsen - nicht unnötig großvolumig zu sein; es muß lediglich gewährleistet werden, daß die Kanüle in das Schutzgehäuse eintauchen kann, wo sie dann komplett eingeschlossen ist. Anschließend kann, je nach Ausführung des Schutzgehäuses, ggf. noch eine Verschlußkappe auf das vordere Gehäuseende aufgesetzt werden.

Es empfiehlt sich, daß die Lage der vollständig in das Schutzgehäuse hineingezogenen Kanüle gesichert wird, indem z.B. im Inneren des Schutzgehäuses eine Klemmung oder Einrastung vorgesehen wird. Eine solche Lagesicherung verhindert, daß sich die in dem Schutzgehäuse eingeschlossene Kanüle selbsttätig aus dem Gehäuse herausbewegt bzw. -fällt, z.B. bei der Bevorratung in einem Sammelgefäß für zu entsorgende Kanülen.

Eine Vorrichtung zum Durchführen des Verfahrens sieht vor, daß in einem eine größere Länge als die Kanüle besitzenden zylindrischen, zumindest einseitig offenen Schutzrohr ein Kanülenhalterkolben angeordnet ist, der an seiner einen Seite mit einem zu der Führungshülse der Kanüle komplementären Adapterkopf und an seiner anderen Seite mit einem aus dem rückwärtigen Schutzrohrende herausragenden Zugelement ausgebildet ist. Es läßt sich somit eine Adaption erreichen, die beim Ankoppeln des Schutzrohres keinen Unterschied gegenüber dem Ankoppeln eines Blutentnahmeröhrchens macht, denn der in die Führungshülse bzw. den Halter der Kanüle eintauchende Adapterkopf entspricht in seiner Funktion und Abmessung denen eines Domes bzw. Ansatzes des das offene Ende eines Blutentnahmeröhrchens verschließenden Stopfens. Es liegt somit der gleiche Kopplungsmechanismus vor, und an der Kanüle selbst braucht nichts verändert zu werden. Sowohl das Blutentnahmeröhrchen als auch die Kanüle bleiben somit unverändert, da die Adaption des Schutzrohres mit zu den bei einer Blutentnahmevorrichtung gleichen Bauteilen vorgenommen wird. Das zum Hereinziehen der Kanüle in das Schutzrohr zu ergreifende Zugelement kann entweder eine herkömmliche Kolbenstange oder beispielsweise auch ein zugfester Faden sein.

Weitere Einzelheiten und Vorteile der Erfindung ergeben sich aus den Ansprüchen und der nachfolgenden Beschreibung, in der ein in den Zeichnungen schematisch dargestelltes Ausführungsbeispiel des Gegenstandes der Erfindung näher erläutert ist. Es zeigen:
Fig. 1 in der Vorder- bzw. Längsansicht ein einer Doppelkanüle zugeordnetes Schutzrohr, vor der Adaption dieser beiden Teile;
Fig. 2 die mit einem in dem Schutzrohr axial beweglich angeordneten Adapterkopf adaptierte Kanüle; und
Fig. 3 die vollständig in das Schutzrohr hineingezogene Kanüle.

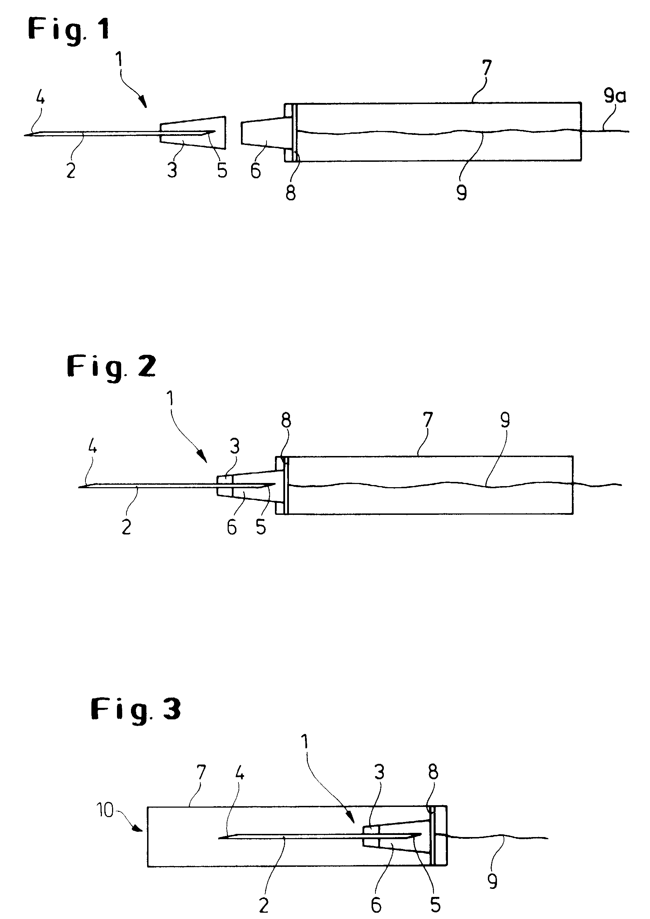

Von einer als solche hinlänglich bekannten Blutentnahmevorrichtung ist in Fig. 1 lediglich die Kanäle 1 dargestellt (schematisch), die aus einer beidseitig angeschärften Nadel 2 und einer diese aufnehmenden Führungshülse 3 besteht. Das mit der vorderen Schneidkante 4 versehene Nadelende ragt frei aus der Führungshülse 3 hervor und ist vor dem Gebrauch der Kanüle 1 mit einer dem Nadeldurchmesser entsprechenden, einen kleinen Innendurchmesser aufweisenden, nicht gezeigten Schutzkappe umhüllt; hingegen wird das die hintere Schneidkante 5 aufweisende Nadelende von der Führungshülse 3 umschlossen. Zur Entsorgung der benutzten Kanüle 1 wird diese über ihre Führungshülse 3 mit einem Adapterkopf 6 eines in einem Schutzrohr 7 axial beweglich angeordneten Kanülenhalterkolbens 8 verbunden. Dies geschieht durch Einschieben des im Ausführungsbeispiel konischen Adapterkopfes, der in dieser Ausgangslage aus dem Schutzrohr 7 hervorragt, in die komplementäre Führungshülse 3 der Kanäle 1; die mit dem Schutzrohr 7 adaptierte Kanüle 1 ist in Figur 2 gezeigt.

An seiner von dem Adapterkopf 6 abgewandten Seite ist der Kanülenhalterkolben 8 mit einem Zugelement 9 verbunden, daß abweichend von dem dargestellten Faden auch eine Kolbenstange sein könnte. Das Zugelement 9 ragt in der Ausgangslage (vgl. die Figuren 1 und 2) mit einem freien Ende 9a rückwärtig bzw. bodenseitig aus dem Schutzrohr 7 hervor; das freie Ende könnte beispielsweise auch eine mit dem Zugelement verbundene, in der in Fig. 1 gezeigten Ausgangslage das Schutzgehäuse 7 hinten übergreifende Kappe oder dergleichen sein. Einem Benutzer ist es somit möglich, das freie Ende 9a des Zugelementes 9 zu ergreifen und den Kanülenhalterkolben 8 zusammen mit der daran angekoppelten Kanüle 1 vollständig in das Schutzrohr 7 hineinzuziehen, so daß die als Adapter benutzte Führungshülse 3 nebst Nadel 2 völlig in dem Schutzrohr eingeschlossen ist (vgl. Fig. 3) und es nicht mehr zu unbeabsichtigten Stichverletzungen kommen kann.

Zur kompletten Einhausung der Kanüle 1 in dem Schutzrohr 2 kann das offene, vordere Schutzrohrende 10 mit einer Schutzkappe (nicht dargestellt) verschlossen werden. Die Kanüle 1 kann dann nicht mehr selbsttätig aus dem Schutzrohr 7 herausfallen; der gleiche Effekt ließe sich durch eine Lagesicherung des Kanülenhalterkolbens 7 in seiner gemäß Fig. 3 gezeigten Hubendlage erreichen, beispielsweise durch Klemmung oder Ausbildung mit entsprechenden Rastmitteln.

## Patentansprüche

1. Verfahren zum Entsorgen der bei einer Blutentnahmevorrichtung zur Blutentnahme mit einem Gefäß adaptierten Kanüle,
**dadurch gekennzeichnet,**
daß die Kanüle nach dem Abkoppeln des Gefäßes mit einem in einem Schutzgehäuse axial beweglichen Zugelement gekoppelt und samt ihres Adapters vollständig in das Schutzgehäuse hineingezogen wird.

2. Vorrichtung zum Durchführen des Verfahrens nach Anspruch 1,
**dadurch gekennzeichnet,**
daß in einem eine größere Länge als die Kanüle (1) besitzenden zylindrischen, zumindest einseitig offenen Schutzrohr (7) ein Kanülenhalterkolben (8) angeordnet ist, der an seiner einen Seite mit einem zu der Führungshülse (3) der Kanüle (1) komplementären Adapterkopf (6) und an seiner anderen Seite mit einem aus dem rückwärtigen Schutzrohrende herausragenden Zugelement (9, 9a) ausgebildet ist.
